(19) Europäisches Patentamt
European Patent Office
Office européen des brevets

(11) Veröffentlichungsnummer: **0 028 765**
**B1**

(12) # EUROPÄISCHE PATENTSCHRIFT

(45) Veröffentlichungstag der Patentschrift: **04.05.83**

(21) Anmeldenummer: **80106612.7**

(22) Anmeldetag: **29.10.80**

(51) Int. Cl.³: **C 07 D 209/08**, C 07 D 215/08, C 07 D 215/12, C 07 D 215/16, C 07 D 241/42, C 07 D 265/36, C 07 D 279/16, A 61 K 31/40, A 61 K 31/47, A 61 K 31/50, A 61 K 31/535

(54) **Alkylharnstoffderivate zur Behandlung von Erkrankungen des Fettstoffwechsels; Verfahren zu deren Herstellung, deren Verwendung in Arzneimitteln zur Behandlung von Fettstoffwechselstörungen, diese enthaltende Arzneimittel, Verfahren zur Herstellung der Arzneimittel sowie einige Alkylharnstoffverbindungen.**

(30) Priorität: **09.11.79 DE 2945238**
**08.08.80 DE 3030024**

(43) Veröffentlichungstag der Anmeldung: **20.05.81 Patentblatt 81/20**

(45) Bekanntmachung des Hinweises auf die Patenterteilung: **04.05.83 Patentblatt 83/18**

(84) Benannte Vertragsstaaten: **AT BE CH DE FR GB IT LI NL SE**

(73) Patentinhaber: **BAYER AG, Zentralbereich Patente, Marken und Lizenzen, D-5090 Leverkusen 1, Bayerwerk (DE)**

(72) Erfinder: **Kabbe, Hans-Joachim, Dr., Walter-Flexstrasse 16, D-5090 Leverkusen (DE)**
Erfinder: **Krause, Hans-Peter, Dr., Wilkhausstrasse 107, D-5600 Wuppertal (DE)**
Erfinder: **Sitt, Rüdiger, Dr., Am Jagd-Haus 116b, D-5600 Wuppertal (DE)**

(56) Entgegenhaltungen:
**DE-A-2 802 630**
**FR-A-2 377 377**
**US-A-3 890 348**
**JOURNAL OF MEDICINAL CHEMISTRY, Band 14, Nr. 1, veröffentlicht im Januar 1971, Washington, US, J. F. MUREN et al.: »Depressant 1,2-Dihydroquinolines and related derivatives«, Seiten 49–53**
**IL FARMACO- Ed. Sc. Band 32, Nr. 4, April 1977, Pavia, IT, M. MAZZA et al.: »Sulla fitotossicita di alcune N′-fenil-N′-alchiluree«, Seiten 270–277**
**IL FARMACO- Ed. Sc., Band 32, Nr. 1, Januar 1977, Pavia, IT, M. MAZZA et al.: »N-acilindoline ad attivita fitotossica. Nota II«, Seiten 54–66**
**IL FARMACO- Ed. Sc. Band 31, Nr. 10, November 1976, Pavia, IT, M. MAZZA et al.: »N-acilinodline ad attivita fitotossica Nota I«, Seiten 746–754**
**JOURNAL OF MEDICINAL CHEMISTRY, Band 12, Nr. 2, März 1969, Washington, US, R. N. PRASAD: »Potential Antihypertensive agents. III 1 3,4-dihydro-2H-1,4-benzothiazine Derivatives«**

**EP 0 028 765 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Alkylharnstoffderivate zur Behandlung von Erkrankungen des Fettstoffwechsels; Verfahren zu deren Herstellung, deren Verwendung in Arzneimitteln zur Behandlung von Fettstoffwechselstörungen, diese enthaltende Arzneimittel, Verfahren zur Herstellung der Arzneimittel sowie einige Alkylharnstoffverbindungen

Die vorliegende Erfindung betrifft die Verwendung von bekannten und neuen Alkylharnstoffderivaten in Arzneimitteln zur Beeinflussung des Fettstoffwechsels, sowie einige neue Verbindungen aus dieser Stoffklasse.

Einige der erfindungsgemäß verwendbaren Alkylharnstoffderivate sind bereits bekannt (vgl. M. Nazza et al., Farmaco, Ed. Sci. 32, No. 1, 54–66 [1977]). Für diese bekannten Verbindungen sind bereits phytotoxische Wirkungen beschrieben. Einige Derivate des Ethoxiquins können auch als Inhibitoren von Eisenvergiftungen verwendet werden (vgl. DE-OS 2 802 630). Ihre Wirkung auf den Fettstoffwechsel, insbesondere ihre lipidabsorptionshemmende Wirkung, ist bisher noch nicht bekannt geworden.

Die vorliegende Erfindung betrifft Alkylharnstoffderivate der allgemeinen Formel (I)

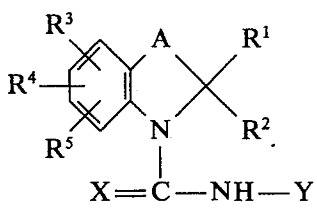

(I)

in welcher

Y für R oder einen Rest

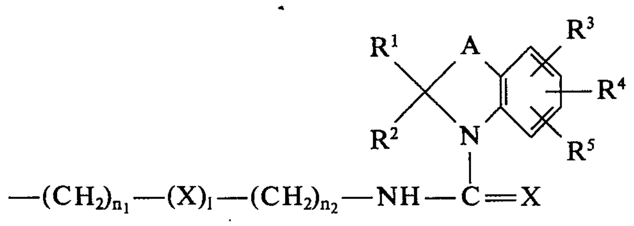

steht, wobei

$n_1$ und $n_2$ ganze Zahlen von 3 bis 9 und
l 0 oder 1 bedeuten und wobei

R für einen geradkettigen, verzweigten, cyclischen, gesättigten oder ungesättigten aliphatischen Kohlenwasserstoffrest mit 4 bis 20 Kohlenstoffatomen steht, der gegebenenfalls durch Halogen, Hydroxy, Alkoxy, oder Alkoxycarbonyl mit jeweils 1 bis 6 Kohlenstoffatomen im Alkoxyrest, oder durch Phenyl substituiert ist, wobei der Phenylrest seinerseits gegebenenfalls 1 oder 2 Substituenten aus der Gruppe Halogen, Trifluormethyl, Hydroxy, Alkyl mit 1 bis 2 Kohlenstoffatomen oder Alkoxy mit 1 bis 2 Kohlenstoffatomen trägt,

$R^1$ für Wasserstoff oder Alkyl mit 1 bis 4 Kohlenstoffatomen steht,

$R^2$ für Alkyl mit 1 bis 4 Kohlenstoffatomen steht,

$R^3$, $R^4$ und $R^5$ gleich oder verschieden sind und jeweils für Wasserstoff, Halogen, Hydroxy, Cyano, Trifluormethyl, Alkyl, Alkylmercapto, Alkylsulfonyl, Alkylsulfinyl, Alkylcarbonyl und Alkoxycarbonyl, wobei die vorgenannten Alkyl- und Alkoxyreste jeweils 1 bis 4 Kohlenstoffatome enthalten, oder für Phenyl oder Phenoxy stehen, wobei die Phenylreste gegebenenfalls 1- oder 2mal substituiert sind durch Halogen, Trifluormethyl oder Alkoxy mit 1 bis 2 Kohlenstoffatomen,

X Sauerstoff oder Schwefel bedeutet und

A für einen der folgenden Reste steht:

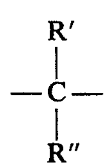 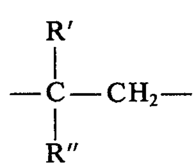 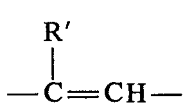 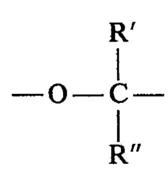 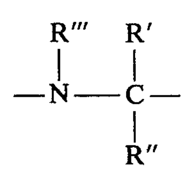

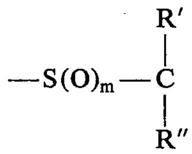

wobei

R' und R'' gleich oder verschieden sind und jeweils Wasserstoff oder Niedrigalkyl bedeuten,
R''' Niedrigalkyl bedeutet und
m 0, 1 oder 2 bedeutet,

zur Verwendung bei der Bekämpfung von Erkrankungen des Fettstoffwechsels sowie bei der Herstellung von fettstoffwechselbeeinflussenden Arzneimitteln sowie einige neue Verbindungen aus dieser Stoffgruppe.

Überraschenderweise zeigen die Alkylharnstoffderivate der allgemeinen Formel (I) eine starke lipidabsorptionshemmende Wirkung. Bei Kenntnis des Standes der Technik konnte nicht erwartet werden, daß Verbindungen dieser Stoffklasse als lipidabsorptionshemmende Wirkstoffe verwendet werden können.

Die erstmalige Verwendung der erfindungsgemäßen Verbindungen bei der Bekämpfung von Hyperlipämien ermöglicht die Behandlung auch solcher Patienten, die gegenüber bereits bekannten Lipidabsorptionshemmern Unverträglichkeit oder Gewöhnung zeigen. Sie stellen somit eine Bereicherung der Pharmazie dar.

Die erfindungsgemäßen Alkylharnstoffderivate der allgemeinen Formel (I) werden in an sich bekannter Weise hergestellt, indem man

a) ein Amin der allgemeinen Formel (II)

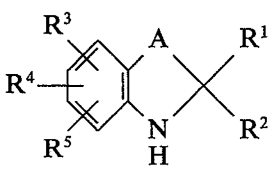

(II)

in welcher

A und $R^1$ bis $R^5$ die oben angegebene Bedeutung haben

mit einer Verbindung der allgemeinen Formel (III)

$$R-N=C=X \qquad (III)$$

in welcher

R und X die oben angegebene Bedeutung haben,

bzw. mit einer Verbindung der allgemeinen Formel (IV)

$$X=C=N-(CH_2)_{n_1}-(X)_l-(CH_2)_{n_2}-N=C=X \qquad (IV)$$

in welcher

X, $n_1$, $n_2$ und l die oben angegebene Bedeutung haben,

gegebenenfalls in einem inerten organischen Lösungsmittel, sowie gegebenenfalls in Anwesenheit an sich bekannter Katalysatoren für Isocyanatreaktionen, bei Temperaturen zwischen 20° und 120° C umsetzt, oder

b) ein Amin der allgemeinen Formel (II) mit Chlorameisensäurephenylester der Formel (V)

$$ClCOOC_6H_5 \qquad (V)$$

bei Temperaturen zwischen 0° und 50° C umsetzt, und den dabei entstandenen Phenylcarbamidsäureester der allgemeinen Formel (VI)

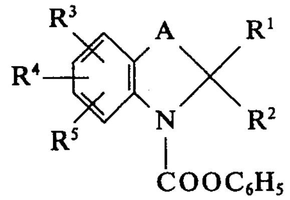

(VI)

in welcher

A und $R^1$ bis $R^5$ die oben angegebene Bedeutung besitzen

direkt oder nach seiner Isolierung mit einem Amin der allgemeinen Formel (VII)

$$H_2N-R \qquad \text{(VII)}$$

bzw. einem Diamin der allgemeinen Formel (VIII)

$$H_2N-(CH_2)_{n_1}-(X)_l-(CH_2)_{n_2}-NH_2 \qquad \text{(VIII)}$$

wobei

R, $n_1$, $n_2$ und l die oben angegebenen Bedeutungen haben,

in einem inerten organischen Lösungsmittel bei Temperaturen zwischen 20 und 140° C umsetzt.

Die vorliegende Erfindung betrifft vorzugsweise die Verwendung von Alkylharnstoffderivaten der allgemeinen Formel (I), in welcher

R für einen geradkettigen, verzweigten, cyclischen, gesättigten oder ungesättigten aliphatischen Kohlenwasserstoffrest mit 4 bis 20 Kohlenstoffatomen steht, der gegebenenfalls durch Halogen, Hydroxy, Alkoxy oder Alkoxycarbonyl mit jeweils 1 bis 6 Kohlenstoffatomen im Alkoxyrest, oder durch Phenyl substituiert ist, wobei der Phenylrest seinerseits gegebenenfalls 1 oder 2 Substituenten aus der Gruppe Halogen, Trifluormethyl, Hydroxy, Alkyl mit 1 bis 2 Kohlenstoffatomen oder Alkoxy mit 1 bis 2 Kohlenstoffatomen trägt,

$R^1$ für Wasserstoff oder Alkyl mit 1 bis 4 Kohlenstoffatomen steht,

$R^2$ für Alkyl mit 1 bis 4 Kohlenstoffatomen steht,

$R^3$, $R^4$ und $R^5$ gleich oder verschieden sind und jeweils für Wasserstoff, Halogen, Hydroxy, Cyano, Trifluormethyl, Alkyl, Alkylmercapto, Alkylsulfonyl, Alkylsulfinyl, Alkylcarbonyl, Alkoxy und Alkoxycarbonyl, wobei die vorgenannten Alkyl- und Alkoxyreste jeweils 1 bis 4 Kohlenstoffatome enthalten, oder für Phenyl oder Phenoxy stehen, wobei die Phenylreste gegebenenfalls 1- oder 2mal substituiert sind durch Halogen, Trifluormethyl oder Alkoxy mit 1 bis 2 Kohlenstoffatomen,

X Sauerstoff oder Schwefel bedeutet und

A für einen der folgenden Reste steht:

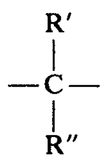 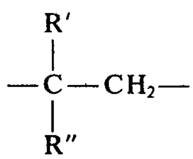 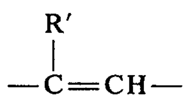 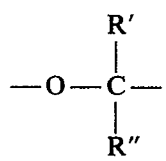 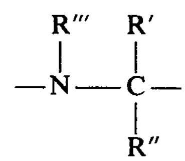

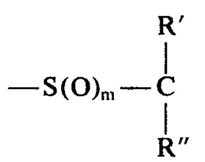

wobei R′ und R″ gleich oder verschieden sind und jeweils Wasserstoff oder Alkyl mit 1 bis 4 Kohlenstoffatomen bedeuten, R‴ Alkyl mit 1 bis 4 Kohlenstoffatomen bedeutet und m für 0 oder 2 steht.

Von besonderem Interesse sind die neuen Alkylharnstoffderivate der allgemeinen Formel (I), in

welcher

R für geradkettiges, verzweigtes oder cyclisches Alkyl oder Alkenyl mit 6 bis 18 Kohlenstoffatomen steht, wobei die genannten Alkyl- und Alkenylgruppen gegebenenfalls substituiert sind durch Chlor, Brom, Fluor oder Alkoxy mit 1 bis 4 Kohlenstoffatomen, oder durch Phenyl,

$R^1$ und $R^2$ gleich oder verschieden sind und jeweils für Alkyl mit 1 oder 2 Kohlenstoffatomen stehen,

$R^3$, $R^4$ und $R^5$ gleich oder verschieden sind und jeweils für Wasserstoff, Chlor, Brom, Trifluormethyl, Alkyl, Alkylmercapto oder Alkoxy stehen, wobei die genannten Alkyl- und Alkoxyreste jeweils 1 bis 4 Kohlenstoffatome enthalten, oder für Phenoxy oder Chlorphenoxy stehen,

A für einen der folgenden Reste steht:

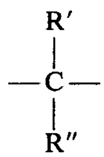 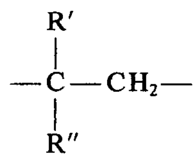 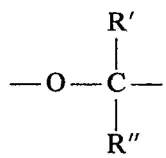 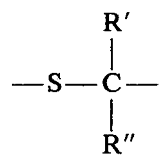

wobei

R′ und R″ gleich oder verschieden sind und jeweils Wasserstoff, Methyl oder Ethyl

bedeuten und

X für Sauerstoff steht.

Von besonderem Interesse sind darüber hinaus auch die neuen Alkylharnstoffderivate der allgemeinen Formel (I), in welcher

Y für einen Rest

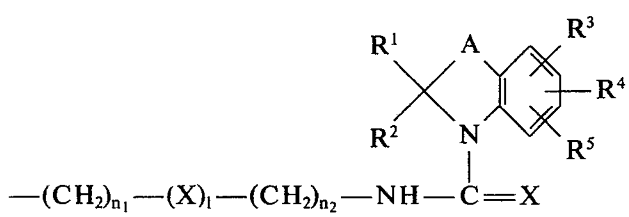

steht, wobei

$R^1$, $R^2$, $R^3$, $R^4$, $R^5$, X, A, $n_1$, $n_2$ und l den obigen Definitionen entsprechen.

Die Herstellung der neuen Verbindungen innerhalb der durch die allgemeine Formel (I) definierten Stoffklasse erfolgt ebenfalls nach an sich bekannten Methoden gemäß den obengenannten Verfahrensvarianten a) und b), wobei die als Ausgangsstoffe verwendeten Isocyanatderivate, Amine und Phenylcarbamidsäureester der allgemeinen Formeln (III), (IV), (VI), (VII) und (VIII) bekannt sind oder nach bekannten Methoden hergestellt werden können [vgl. R. Wagner et. al., Synthetic Organic Chemistry, Wiley, New York (1953), S. 640, 645, 653 und Beilstein, Band XX, 2. Ergänzungswerk, S. 180, 192, 210].

Die Verbindungen der allgemeinen Formel (I) zeigen eine vorteilhafte Hemmung der Lipidabsorption bei Mensch und Tier. Bei der Aufnahme fetthaltiger Nahrung führen sie zu einer geringeren alimentären Hyperlipämie, bei gleichzeitiger Hemmung der Cholesterinabsorption, so daß sie insbesondere zur Behandlung von Fettstoffwechselstörungen, wie z. B. Hyperlipoproteinämien, Atherosklerose oder Adipositas verwendet werden können.

Der Nachweis der vorteilhaften Wirkung läßt sich durch folgende Versuchsanordnung an Ratten zeigen:

Zur Erzeugung einer alimentären Hyperlipämie erhält eine Gruppe von Ratten 2,5 ml/kg Olivenöl per os verabreicht (Kontrollgruppe). Eine entsprechende Gruppe von anderen Ratten erhält gleichzeitig mit der Olivenölapplikation die Wirksubstanz als Suspension in Traganthschleim mit der Schlundsonde verabreicht. Eine weitere Kontrollgruppe von Ratten erhält nur Traganthschleim appliziert.

2 Stunden nach der Applikation von Olivenöl werden die Konzentrationen der Serumtriglyceride in allen drei Rattengruppen bestimmt (Methode: J. Ziegenhorst, Klin. Chem. 21 [1975] 1627). Zwei Stunden nach der Fettapplikation zeigen die nur mit Olivenöl behandelten Ratten (Gruppe 1) gegenüber den Ratten ohne Fettapplikation (Gruppe 3) einen deutlichen Anstieg der Serumtriglyceride. Mit diesem Anstieg, der gleich 100% gesetzt wird, werden die verminderten Serumtriglycerid-Anstiege der mit Wirksubstanz und Olivenöl behandelten Tiere (Gruppe 2) verglichen. Es wurde gefunden, daß bereits geringe Dosierungen der Harnstoffderivate gemäß Formel (I) eine signifikante Senkung der Serumtriglyceride verursachen. Neben der starken lipidabsorptionshemmenden Wirkung zeigen die Verbindungen auch eine ausgesprochen gute Verträglichkeit.

Zur vorliegenden Erfindung gehören pharmazeutische Zubereitungen, die neben nichttoxischen, inerten pharmazeutisch geeigneten Trägerstoffen eine oder mehrere Verbindungen der obigen Formel enthalten oder die aus einer oder mehreren Verbindungen der obigen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen, vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder $^1/_2$, $^1/_3$ oder $^1/_4$ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Lösungen, Suspensionen und Emulsionen und Pasten genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quarternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum-, Calcium- und Magnesiumstearat und feste Polyäthylenglykole oder Gemische der unter (a)–(i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Lösungen und Emulsionen können neben dem oder den Wirkstoffen die üblichen Trägerstoffe, wie Lösungsmittel, Lösungsvermittler und Emulgatoren, z. B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z. B. Wasser, Äthylalkohol, Propylenglykol, Suspendiermittel, z. B. äthoxylierte Isostearylalkohole, Polyoxyäthylensorbit- und -sorbitanester, mikrokristalline Cellulose, Aluminiummethanhydroxid, Bentonit, Agar-Agar und Traganth oder Gemische dieser Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl, und Süßmittel, z. B. Saccharin, enthalten.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gewichtsprozent der Gesamtmischung, vorhanden sein.

Die oben aufgeführten pharmazeutischen Zubereitungen können außer Verbindungen der obigen Formel auch andere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehört auch die Verwendung der Verbindungen der obigen Formel sowie die Verwendung von pharmazeutischen Zubereitungen, die eine oder mehrere Verbindungen

der oben angegebenen Formel enthalten, in der Human- und Veterinärmedizin zur Verhütung, Besserung und/oder Heilung von Erkrankungen des Fettstoffwechsels.

Die Wirkstoffe oder die pharmazeutischen Zubereitungen können oral oder parenteral, vorzugsweise oral, appliziert werden.

Im allgemeinen hat es sich sowohl in der Human- als auch in der Veterinär-Medizin als vorteilhaft erwiesen, den oder die Wirkstoffe in Mengen von etwa 0,1 bis etwa 100, vorzugsweise 1 bis 50 mg/kg Körpergewicht je 24 Stunden, verteilt auf 1 bis 6 Verabreichungen, und zwar vor und/oder während und/oder nach der Mahlzeit zu applizieren. Eine Einzelgabe enthält den oder die Wirkstoffe vorzugsweise in Mengen von etwa 0,1 bis etwa 20 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der o. g. Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die folgenden Formulierungsbeispiele erläutern die Herstellung von erfindungsgemäß zu verwendenden Arzneimittelzubereitungen:

Beispiele für Tablettenherstellung

1. 100 mg der Verbindung des Beispiels 1 mit 69 mg Milchzucker und 30 mg Maisstärke gemischt, anschließend mit einem Kleister aus 15 mg Maisstärke angeknetet und durch ein Sieb mit 3—5 mm Maschenweite gedrückt. Anschließend wird in einem Trockner bei 60—80° C getrocknet. Das erhaltene Granulat wird durch ein Sieb mit 0,8 mm Maschenweite geschlagen, weitere 15 mg Maisstärke, 10 mg Talkum und 1 mg Magnesiumstearat werden zugemischt und mit Hilfe einer üblichen Tablettenpresse zu runden Tabletten mit 9 mm Durchmesser und einem Gesamtgewicht von 240 mg verpreßt.
2. 200 mg der Verbindung des Beispiels 13 werden mit 97 mg sekundärem Calciumphosphat vermicht und mit einer wäßrigen Gelatinelösung, die 2 mg Gelatine enthält, angeknetet. Anschließend wird durch ein Sieb mit 3—5 mm Maschenweite gedrückt und bei 60—80°C getrocknet. Das trockene Granulat wird gesiebt (0,8 mm), anschließend 20 mg Weizenstärke und 1 mg Magnesiumstearat zugemischt und auf bekannte Weise tablettiert. Man erhält runde Tabletten vom Durchmesser 8 mm und einem Gesamtgewicht von 320 mg.

Von besonderem Interesse sind die neuen Alkylharnstoffderivate der allgemeinen Formel (I), in welcher die Substituenten die folgende Bedeutung besitzen:

R bedeutet geradkettiges, verzweigtes oder cyclisches Alkyl oder Alkenyl mit jeweils 6 bis 18 Kohlenstoffatomen, gegebenenfalls substituiert durch Chlor, Brom, Fluor oder Alkoxy mit 1 bis 4 Kohlenstoffatomen,

$R^1$ und $R^2$ bedeuten Methyl oder Ethyl,

$R^3$, $R^4$ und $R^5$ bedeuten Wasserstoff, Chlor, Brom, Trifluormethyl, Phenoxy, Chlorphenoxy, Alkyl, Mercaptoalkyl oder Alkoxy mit jeweils 1 bis 2 Kohlenstoffatomen,

X bedeutet Sauerstoff und

A steht für einen der folgenden Reste:

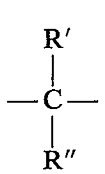   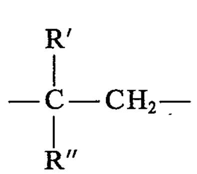   $-OCH_2-$   $-SCH_2-$

wobei

R′ und R″ jeweils für Wasserstoff, Methyl oder Ethyl stehen.

Als neue Verbindungen seien beispielsweise genannt:

N-n-Hexylaminocarbonyl-2,2,4-trimethyl-1,2,3,4-tetrahydrochinolin,
N-n-Octylaminocarbonyl-2,2,4-trimethyl-1,2,3,4-tetrahydrochinolin,
N-2-Ethylhexylaminocarbonyl-2,2,4-trimethyl-1,2,3,4-tetrahydrochinolin,
N-n-Tetradecylaminocarbonyl-2,2,4-trimethyl-1,2,3,4-tetrahydrochinolin,

N-3-n-Butoxypropylaminocarbonyl-2,2,4-trimethyl-1,2,3,4-tetrahydrochinolin,
N-n-Hexylaminocarbonyl-2-methylindolin,
N-n-Hexylaminocarbonyl-2,2-dimethylindolin,
N-n-Dodecylaminocarbonyl-2,2-dimethylindolin,
N-n-Octylaminocarbonyl-2,2,4-trimethyl-6-phenoxy-1,2,3,4-tetrahydrochinolin,
N-n-Octylaminocarbonyl-2,2,4-trimethyl-6-butoxy-1,2,3,4-tetrahydrochinolin,
6,6'-Bis-(N-2,2,4-trimethyl-1,2,3,4-tetrahydro-chinolyl)-di-n-hexylether und
6,6'-Bis-(N-2,2,4-trimethyl-1,2,3,4-tetrahydro-chinolyl)-di-n-butylether.

## Herstellungsbeispiele

### Beispiel 1
(Variante a)

0,1 Mol 2.2.4-Trimethyl-1.2.3-4-tetrahydrochinolin werden in 50 ml Ether gelöst und mit 0,1 Mol n-Dodecylisocyanat vermicht. Anschließend versetzt man mit einer Spatelspitze Diazabicyclooctan, läßt 7 Tage bei 20 bis 25° C stehen, kühlt auf −70° C ab und saugt den ausgefallenen Niederschlag ab. Man erhält N-n-Dodecylaminocarbonyl-2.2.4-trimethyl-1.2.3.4-tetrahydrochinolin vom Schmelzpunkt 48−50° C.
Ausbeute: 66% der Theorie.

### Beispiel 2
(Variante a)

1 Mol 2-Methylindolin wird in 300 ml Toluol gelöst und mit 1 Mol Methylisocyanat versetzt, wobei die Temperatur nicht über 25° C ansteigt. Man läßt 1 Tag stehen und saugt dann den erhaltenen Niederschlag ab. Man erhält N-Methylaminocarbonyl-2-methylindolin vom Schmelzpunkt 158−160° C.
Ausbeute: 89% der Theorie.

### Beispiel 3
(Variante b)

Eine Lösung von 0,1 Mol 2-Methylindolin, 0,12 Mol Triethylamin, 100 ml Toluol und 0,1 Mol Chlorameisensäurephenylester wird 10 Stunden bei 40° C gehalten, anschließend auf 20° C abgekühlt und mit 250 ml Wasser 15 Minuten verrührt. Man trennt die Toluolphase ab, verdampft das Lösungsmittel unter Vakuum, löst den Rückstand in 100 ml n-Hexylamin auf und erhitzt diese Lösung 6 Stunden unter Rückfluß auf 130° C. Nach Abkühlen wird das Lösungsmittel bei 50° C/10 Torr abgedampft und der Rückstand in 250 ml Toluol aufgenommen. Anschließend wird die Lösung nacheinander mit jeweils 200 ml 1n-Natronlauge, 1n-Salzsäure und Wasser extrahiert. Nach Einengen der Lösung wird der Rückstand aus Petrolether umkristallisiert. Man erhält N-n-Hexyl-aminocarbonyl-2-methylindolin vom Schmelzpunkt 57−59° C.
Ausbeute: 53% der Theorie.

Analog Beispiel 1 werden die Verbindungen der folgenden Tabelle 1 erhalten:

Tabelle 1

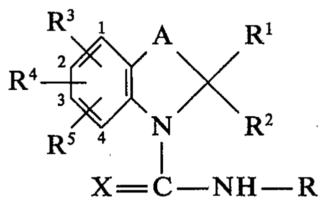

| Beispiel Nr. | A | X | R | $R^1$ | $R^2$ | $R^3$, $R^4$, $R^5$ | Fp (°C) | Ausbeute in % d.Th. | Bemerkung |
|---|---|---|---|---|---|---|---|---|---|
| 4 | $-S-CH_2-$ | O | n-$C_{12}H_{25}$ | $CH_3$ | $CH_3$ | H | 102–104 | 24 | |
| 5 | 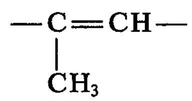 | O | n-$C_{12}H_{25}$ | $CH_3$ | $CH_3$ | H | 70– 71 | 36 | |
| 6 | 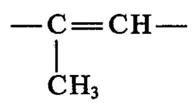 | O | n-$C_{12}H_{25}$ | $CH_3$ | $CH_3$ | 2-Cl | | 94 | Öl |
| 7 | 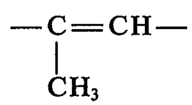 | O | n-$C_{12}H_{25}$ | $CH_3$ | $CH_3$ | 2-$OC_2H_5$ | 56– 57 | 71 | |
| 8 | 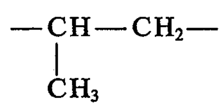 | O | n-$C_{12}H_{25}$ | $CH_3$ | $CH_3$ | 2-$OC_2H_5$ | | 98 | Öl |
| 9 | 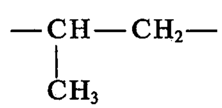 | O | n-$C_{16}H_{33}$ | $CH_3$ | $CH_3$ | H | 62– 63 | 41 | |
| 10 | 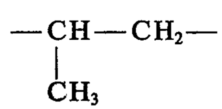 | O | n-$C_{18}H_{27}$ | $CH_3$ | $CH_3$ | H | 62– 64 | 35 | |
| 11 | 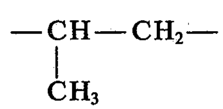 | O | $CH_3$ | $CH_3$ | $CH_3$ | H | 105–107 | 77 | |
| 12 | 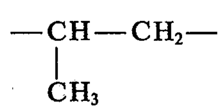 | O | iso-$C_3H_7$ | $CH_3$ | $CH_3$ | H | 79– 81 | 31 | |

Tabelle 1 (Fortsetzung)

| Beispiel Nr. | A | X | R | $R^1$ | $R^2$ | $R^3$, $R^4$, $R^5$ | Fp (°C) | Ausbeute in % d.Th. | Bemerkung |
|---|---|---|---|---|---|---|---|---|---|
| 13 | $-CH(CH_3)-CH_2-$ | O | $(CH_2)_6-Cl$ | $CH_3$ | $CH_3$ | H | 71 - 73 | 68 | |
| 14 | $-CH(CH_3)-CH_2-$ | O | n-$C_4H_9$ | $CH_3$ | $CH_3$ | H | 48 - 50 | 54 | |
| 15 | $-CH(CH_3)-CH_2-$ | O | i-$C_4H_9$ | $CH_3$ | $CH_3$ | H | 68 - 70 | 42 | |
| 16 | $-CH_2-$ | O | iso-$C_3H_7$ | H | $CH_3$ | H | 129 - 131 | 92 | |
| 17 | $-CH_2-$ | O | n-$C_4H_9$ | H | $CH_3$ | H | 99 - 101 | 73 | |
| 18 | $-CH_2-$ | O | tert.-$C_4H_9$ | H | $CH_3$ | H | 133 - 135 | 76 | |
| 19 | $-CH_2-$ | O | $(CH_2)_6-Cl$ | H | $CH_3$ | H | 74 - 76 | 89 | |
| 20 | $-CH_2-$ | O | n-$C_{12}H_{25}$ | H | $CH_3$ | H | 78 - 80 | 91 | |
| 21 | $-CH_2-$ | S | Allyl | H | $CH_3$ | H | | 98 | Öl |

Beispiel 22

Eine Lösung von 35 g 2,2,4-Trimethyl-1,2,3,4-tetrahydrochinolin in 100 ml Ether wird mit 16,8 g Hexamethylendiisocyanat versetzt. Nach 1 Tag gibt man 1/2 g DABCO (Diazabicyclooctan) hinzu, läßt 3 Tage stehen und saugt 23,8 g 1,6-Bis-(N-2,2,4-trimethyl-1,2,3,4-tetrahydrochinolyl)-carbonylamino-hexan ab. Aus der Mutterlauge erhält man weitere 25,1 g (Gesamtausbeute: 48,1 g = 94%); Schmp. 125 – 128° C.

Beispiel 23

Eine Lösung von 35 g 2,2,4-Trimethyl-1,2,3,4-tetrahydrochinolin in 100 ml abs. Tetrahydrofuran wird mit 18,2 g 1,7-Diisocyanatoheptan versetzt. Nach 1 Tag gibt man 1/2 g DABCO hinzu. Nach längerem Stehen filtriert man von etwas Ungelöstem ab, engt das Filtrat ein, löst den Rückstand in ca. 100 ml Ether, verdünnt die Lösung mit 200 ml Petrolether, kühlt auf –70° C ab und saugt nach 1 Tag ab. Ausbeute an 1,7-Bis-(N-2,2,4-trimethyl-1,2,3,4-tetrahydrochinolyl)-carbonylamino-heptan: 42,9 g; Schmp. 112 – 116° C.

Beispiel 24

Eine Lösung von 35 g 2,2,4-Trimethyl-1,2,3,4-tetrahydrochinolin, 23,8 g 1,11-Diisocyanatoundecan und 1/2 g DABCO läßt man 3 Tage stehen, versetzt man 150 ml Toluol/Petrolether (1 : 1), erhitzt kurz zum Sieden und saugt ab. Ausbeute: 46,4 g (83%) 1,11-Bis-(N-2,2,4-trimethyl-1,2,3,4-tetrahydrochinolyl)-carbonylaminoundecan; Schmp. 102 – 108° C.

Die weiteren Produkte wurden analog zu Beispiel 22, 23 bzw. 24 hergestellt (siehe die folgende Tabelle 2, in welcher die Verfahrensprodukte entsprechend der allgemeinen Formel

$$Q-\underset{\underset{O}{\|}}{C}-NH-(CH_2)_{n_1}-(S)_l-(CH_2)_{n_2}-NH-\underset{\underset{O}{\|}}{C}-Q$$

definiert sind).

Tabelle 2

| Beispiel | Q | $n_1$ | $n_2$ | 1 | Ausbeute (% d. Theorie) | Verfahrensweise analog Beispiel | Schmelzpunkt (°C) |
|---|---|---|---|---|---|---|---|
| 25 | 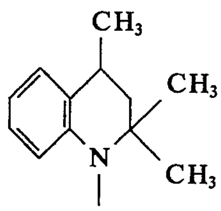 | 4 | 4 | 0 | 48 | 23 | 93–98 |
| 26 | desgl. | 6 | 6 | 1 | 42 | 24 | 78–79 |
| 27 | 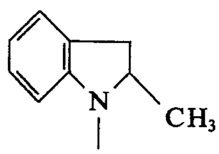 | 6 | 5 | 0 | 80 | 23 | 66–68 |
| 28 | desgl. | 3 | 3 | 0 | 95 | 23 | 158–60 |
| 29 | 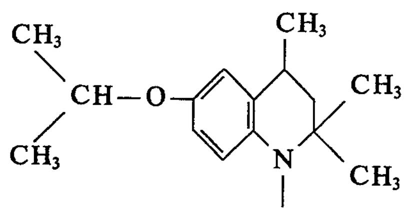 | 6 | 5 | 0 | 39 | 22 | 64–68 |
| 30 | 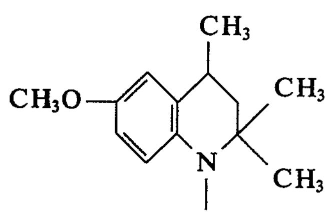 | 6 | 5 | 0 | 52 | 22 | 81–84 |

Analog zu Beispiel 1 wurden die Verbindungen der folgenden Tabelle 3 erhalten, wobei A, X, R, $R^1$, $R^2$ und $R^3$ gemäß Tabelle 1 definiert sind.

Tabelle 3

| Beispiel-Nr. | A | X | R | $R^1$ | $R^2$ | $R^3$ | Fp (°C) | Ausbeute (in % d.Th.) |
|---|---|---|---|---|---|---|---|---|
| 31 | $-\overset{CH_3}{\overset{\vert}{C}H}-CH_2-$ | O | $CH_2-CF_3$ | $CH_3$ | $CH_3$ | H | 120–122°C | 86 |
| 32 | $-CH_2-CH_2-$ | O | Cyclohexyl | $CH_3$ | H | H | 57– 59°C | 66 |
| 33 | $-CH_2-CH_2-$ | O | n-$C_{12}H_{25}$ | $CH_3$ | H | H | 44– 46°C | 74 |
| 34 | $-CH_2-CH_2$ | O | $CH_3$ | $CH_3$ | H | H | 70– 72°C | 82 |
| 35 | $-C(CH_3)=CH-$ | O | Cyclohexyl | $CH_3$ | $CH_3$ | 2-$OC_2H_5$ | 135–137°C | 79 |
| 36 | $-\underset{CH_3}{\underset{\vert}{C}H}-CH_2-$ | O | $CH_2-CH_2-COO-$<br>$C_3H_7$ | $CH_3$ | $CH_3$ | H | Öl | 90 |
| 37 | $-\underset{CH_3}{\underset{\vert}{C}H}-CH_2-$ | O | $(CH_2)_5-COO-$<br>$CH_3$ | $CH_3$ | $CH_3$ | H | Öl | 90 |
| 38 | $-CH_2-CH_2-$ | O | $CH_3$ | $CH_3$ | H | $CH_3$ | 128–130°C | 64 |
| 39 | $-CH_2-CH_2$ | O | n-$C_{12}H_{25}$ | $CH_3$ | H | $CH_3$ | 70– 72°C | 57 |

**Patentansprüche**

1. Alkylharnstoffe der allgemeinen Formel (I)

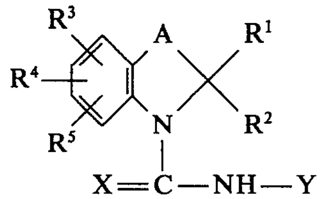

(I)

in welcher

Y für R oder einen Rest

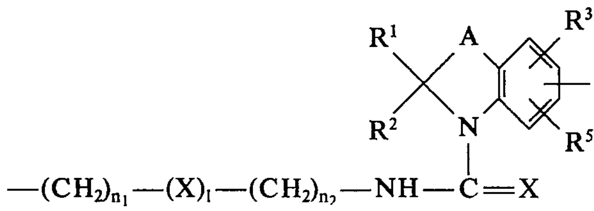

steht, wobei

$n_1$ und $n_2$ ganze Zahlen von 3 bis 9 und
I 0 oder 1

bedeuten und wobei

R für einen geradkettigen, verzweigten, cyclischen, gesättigten oder ungesättigten aliphatischen Kohlenwasserstoffrest mit 4 bis 20 Kohlenstoffatomen steht, der gegebenenfalls durch Halogen, Hydroxy, Alkoxy, oder Alkoxycarbonyl mit jeweils 1 bis 6 Kohlenstoffatomen im Alkoxyrest, oder durch Phenyl substituiert ist, wobei der Phenylrest seinerseits gegebenenfalls 1 oder 2 Substituenten aus der Gruppe Halogen, Trifluormethyl, Hydroxy, Alkyl mit 1 bis 2 Kohlenstoffatomen oder Alkoxy mit 1 bis 2 Kohlenstoffatomen trägt,
$R^1$ für Wasserstoff oder Alkyl mit 1 bis 4 Kohlenstoffatomen steht,
$R^2$ für Alkyl mit 1 bis 4 Kohlenstoffatomen steht,
$R^3$, $R^4$ und $R^5$ gleich oder verschieden sind und jeweils für Wasserstoff, Halogen, Hydroxy, Cyano, Trifluormethyl, Alkyl, Alkylmercapto, Alkylsulfonyl, Alkylsulfinyl, Alkylcarbonyl und Alkoxycarbonyl, wobei die vorgenannten Alkyl- und Alkoxyreste jeweils 1 bis 4 Kohlenstoffatome enthalten, oder für Phenyl oder Phenoxy stehen, wobei die Phenylreste gegebenenfalls 1- oder 2mal substituiert sind durch Halogen, Trifluormethyl oder Alkoxy mit 1 bis 2 Kohlenstoffatomen,
X Sauerstoff oder Schwefel bedeutet und
A für einen der folgenden Reste steht:

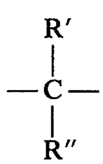 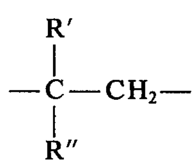 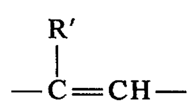 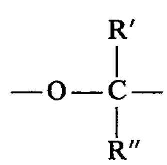 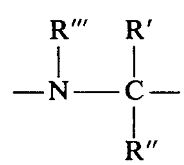

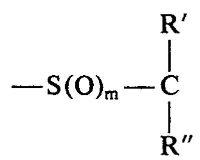

wobei

R′ und R″ gleich oder verschieden sind und jeweils Wasserstoff oder Alkyl mit 1 bis 4 Kohlenstoffatomen bedeuten,

R''' Alkyl mit 1 bis 4 Kohlenstoffatomen bedeutet und
m für 0 oder 2 steht,

zur Verwendung bei der Bekämpfung von Erkrankungen des Fettstoffwechsels.

2. Alkylharnstoffderivate der allgemeinen Formel (I) von Anspruch 1, in welcher

R für geradkettiges, verzweigtes oder cyclisches Alkyl oder Alkenyl mit 6 bis 18 Kohlenstoffatomen steht, wobei die genannten Alkyl- und Alkenylgruppen gegebenenfalls substituiert sind durch Chlor, Brom, Fluor oder Alkoxy mit 1 bis 4 Kohlenstoffatomen oder durch Phenyl,
$R^1$ und $R^2$ gleich oder verschieden sind und jeweils für Alkyl mit 1 oder 2 Kohlenstoffatomen stehen,
$R^3$, $R^4$ und $R^5$ gleich oder verschieden sind und jeweils für Wasserstoff, Chlor, Brom, Trifluormethyl, Alkyl oder Alkylmercapto stehen, wobei die genannten Alkylreste jeweils 1 bis 4 Kohlenstoffatome enthalten, oder für Phenoxy oder Chlorphenoxy stehen,
A für einen der folgenden Reste steht:

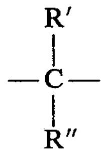 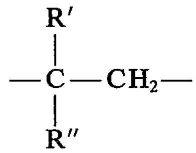 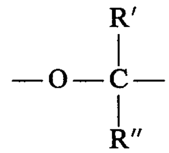 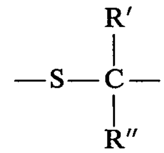

wobei

R' und R'' gleich oder verschieden sind und jeweils Wasserstoff, Methyl oder Ethyl

bedeuten und

X für Sauerstoff steht.

3. Alkylharnstoffderivate nach Anspruch 2, dadurch gekennzeichnet, daß

R für geradkettiges, verzweigtes oder cyclisches Alkyl oder Alkenyl mit jeweils 6 bis 18 Kohlenstoffatomen steht, welches gegebenenfalls substituiert ist durch Chlor, Brom, Fluor oder Alkoxy mit 1 bis 4 Kohlenstoffatomen,
$R^1$ und $R^2$ gleich oder verschieden sind und jeweils für Methyl oder Ethyl stehen,
$R^3$, $R^4$ und $R^5$ gleich oder verschieden sind und jeweils für Wasserstoff, Chlor, Brom, Trifluormethyl, Phenoxy, Chlorphenoxy, Alkyl oder Mercaptoalkyl mit jeweils 1 bis 2 Kohlenstoffatomen stehen,
X für Sauerstoff steht und
A für einen der folgenden Reste steht:

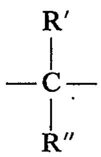 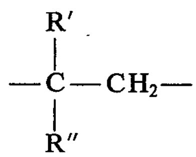 $—OCH_2—$ $—SCH_2—$

wobei

R' und R'' jeweils Methyl oder Ethyl

bedeuten.

4. N-n-Dodecylaminocarbonyl-2,2,4-trimethyl-1,2,3,4-tetrahydrochinolin.

5. Alkylharnstoffderivate der allgemeinen Formel (I) von Anspruch 1, in welcher

Y für einen Rest

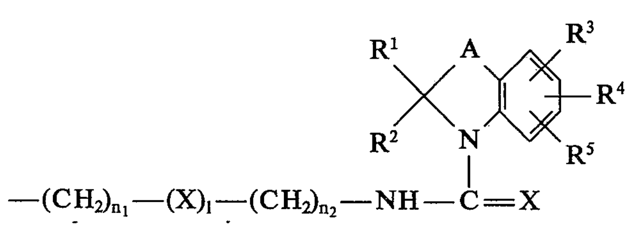

steht, wobei

| | |
|---|---|
| $n_1$ und $n_2$ | ganze Zahlen von 3 bis 9 und |
| l | 0 oder 1 bedeuten, und |
| $R^1$, $R^2$, $R^3$, $R^4$, $R^5$, A und X | die in den Ansprüchen 2 oder 3 angegebene Bedeutung haben. |

6. Verfahren zur Herstellung von Verbindungen der allgemeinen Formel (I) gemäß Ansprüchen 1und 3, dadurch gekennzeichnet, daß man

a) ein Amin der allgemeinen Formel (II),

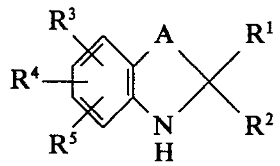

(II)

in welcher

| | |
|---|---|
| A und $R^1$ bis $R^5$ | die oben angegebene Bedeutung haben |

mit einer Verbindung der allgemeinen Formel (III)

$$R-N=C=X \qquad (III)$$

bzw. einer Verbindung der allgemeinen Formel (IV)

$$X=C=N-(CH_2)_{n_1}-(X)_l-(CH_2)_{n_2}-N=C=X \qquad (IV)$$

wobei

| | |
|---|---|
| R, X, $n_1$, $n_2$ und l | die oben angegebene Bedeutung haben, |

gegebenenfalls in einem inerten organischen Lösungsmittel, sowie gegebenenfalls in Anwesenheit eines Katalysators, bei Temperaturen zwischen 20° C und 120° C umsetzt, oder

b) ein Amin der allgemeinen Formel (II) mit Chlorameisensäurephenylester der Formel (V)

$$ClCOOC_6H_5 \qquad (V)$$

bei Temperaturen zwischen 0° C und 50° C umsetzt, und den dabei entstandenen Phenylcarbamidsäureester der allgemeinen Formel (VI)

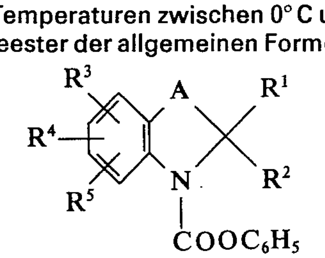

(VI)

in welcher

| | |
|---|---|
| A und $R^1$ bis $R^5$ | die oben angegebene Bedeutung besitzen, |

direkt oder nach seiner Isolierung mit einem Amin der allgemeinen Formel (VII)

$$H_2N-R \qquad (VII)$$

bzw. einem Diamin der allgemeinen Formel (VIII)

$$H_2N-(CH_2)_{n_1}-(X)_l-(CH_2)_{n_2}-NH_2 \qquad (VIII)$$

wobei

| | |
|---|---|
| R, $n_1$, $n_2$ und l | die oben angegebenen Bedeutungen haben, |

in einem inerten organischen Lösungsmittel bei Temperaturen zwischen 20 und 140° C umsetzt.

7. Fettstoffwechsel beeinflussende Arzneimittel, enthaltend mindestens eine Verbindung gemäß Anspruch 1 oder 2.

8. Verfahren zur Herstellung von fettstoffwechselbeeinflussenden Arzneimitteln, dadurch gekennzeichnet, daß man Verbindungen gemäß Anspruch 1 oder 2, gegebenenfalls unter Verwendung üblicher Hilfs- und Trägerstoffe, in eine geeignete Applikationsform überführt.

9. Verbindungen der allgemeinen Formel (I) gemäß Anspruch 1 zur Verwendung bei der Herstellung von Arzneimitteln.

10. Verbindungen der allgemeinen Formel (I) gemäß Anspruch 1 zur Verwendung bei der Bekämpfung von Hyperlipidämie.

**Claims**

1. Alkylureas of the general formula (I)

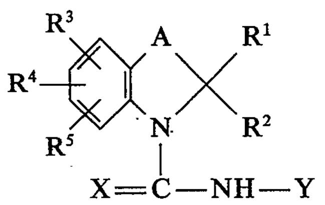

(I)

in which

Y represents R or a radical

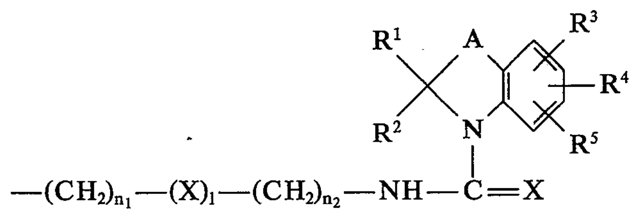

wherein

$n_1$ and $n_2$ denote integers from 3 to 9 and
l denotes 0 or 1 and

wherein

R represents a straight-chain, branched, cyclic, saturated or unsaturated aliphatic hydrocarbon radical with 4 to 20 carbon atoms, which is optionally substituted by halogen, hydroxyl, alkoxy or alkoxycarbonyl with, in each case, 1 to 6 carbon atoms in the alkoxy radical, or by phenyl, the phenyl radical in turn optionally carrying 1 or 2 substituents from the group comprising halogen, trifluoromethyl, hydroxyl, alkyl with 1 to 2 carbon atoms or alkoxy with 1 to 2 carbon atoms,

$R^1$ represents hydrogen or alkyl with 1 to 4 carbon atoms,

$R^2$ represents alkyl with 1 to 4 carbon atoms,

$R^3$, $R^4$ and $R^5$ are identical or different and each represent hydrogen, halogen, hydroxyl, cyano, trifluoromethyl, alkyl, alkylmercapto, alkylsulphonyl, alkylsulphinyl, alkylcarbonyl and alkoxycarbonyl, the abovementioned alkyl and alkoxy radicals containing 1 to 4 carbon atoms in each case, or represent phenyl or phenoxy, the phenyl radicals optionally being monosubstituted or disubstituted by halogen, trifluoromethyl or alkoxy with 1 to 2 carbon atoms,

X denotes oxygen or sulphur and

A represents one of the following radicals:

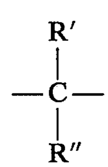 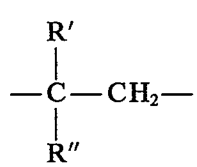 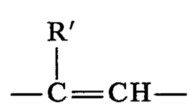 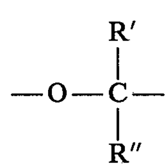 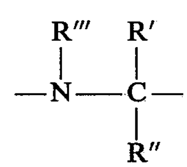

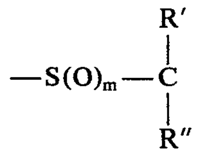

wherein

R′ and R″ are identical or different and each denote hydrogen or alkyl with 1 to 4 carbon atoms,
R‴ denotes alkyl with 1 to 4 carbon atoms and
m represents 0 or 2,

for use in combating diseases of the lipometabolism.

2. Alkylurea derivatives of the general formula (I) of Claim 1, in which

R represents straight-chain, branched or cyclic alkyl or alkenyl with 6 to 18 carbon atoms, the said alkyl and alkenyl groups optionally being substituted by chlorine, bromine, fluorine or alkoxy with 1 to 4 carbon atoms or by phenyl,
$R^1$ and $R^2$ are identical or different and each represent alkyl with 1 or 2 carbon atoms,
$R^3$, $R^4$ and $R^5$ are identical or different and each represent hydrogen, chlorine, bromine, trifluoromethyl, alkyl or alkylmercapto, the said alkyl radicals each containing 1 to 4 carbon atoms, or represent phenoxy or chlorophenoxy,
A represents one of the following radicals:

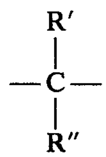 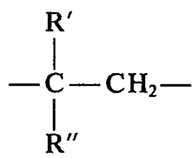 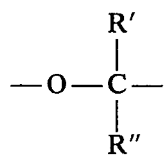 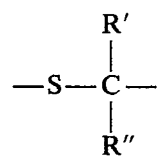

wherein

R′ and R″ are identical or different and each denote hydrogen, methyl or ethyl and
X represents oxygen.

3. Alkylurea derivatives according to Claim 2, characterised in that

R represents straight-chain, branched or cyclic alkyl or alkenyl with in each case 6 to 18 carbon atoms, which is optionally substituted by chlorine, bromine, fluorine or alkoxy with 1 to 4 carbon atoms,
$R^1$ and $R^2$ are identical or different and each represent methyl or ethyl,
$R^3$, $R^4$ and $R^5$ are identical or different and each represent hydrogen, chlorine, bromine, trifluoromethyl, phenoxy, chlorophenoxy, alkyl or alkylmercapto with in each case 1 to 2 carbon atoms,
X represents oxygen and
A represents one of the following radicals:

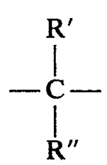 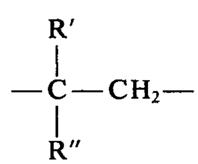 $-OCH_2-$ $-SCH_2-$

wherein

R′ and R″ each denote methyl or ethyl.

4. N-n-Dodecylaminocarbonyl-2,2,4-trimethyl-1,2,3,4-tetrahydroquinoline.

5. Alkylurea derivatives of the general formula (I) of Claim 1, in which

Y represents a radical

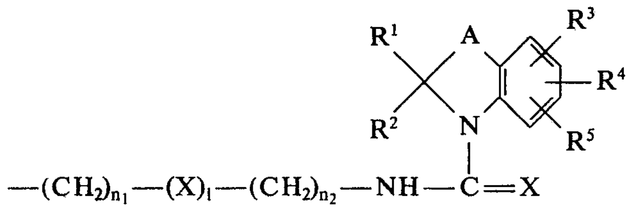

in which

| | |
|---|---|
| $n_1$ and $n_2$ | denote integers from 3 to 9 and |
| l | denotes 0 or 1, and |
| $R^1$, $R^2$, $R^3$, $R^4$, $R^5$, A and X | have the meaning indicated in Claims 2 or 3. |

6. Process for the preparation of compounds of the general formula (I) according to Claims 1 and 3, characterised in that

a) an amine of the general formula (II)

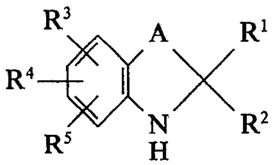

(II)

in which

A and $R^1$ to $R^5$ have the abovementioned meaning,

is reacted with a compound of the general formula (III)

$$R-N=C=X \quad (III)$$

or with a compound of the general formula (IV)

$$X=C=N-(CH_2)_{n1}-(X)_l-(CH_2)_{n2}-N=C=X \quad (IV)$$

in which

R, X, $n_1$, $n_2$ and l have the abovementioned meaning,

optionally in an inert organic solvent and optionally in the presence of a catalyst, at temperatures between 20° an and 120° C, or

b) an amine of the general formula (II) is reacted with phenyl chloroformate of the formula (V)

$$ClCOOC_6H_5 \quad (V)$$

at temperatures between 0° and 50° C and the resulting phenylcarbamic acid ester of the general formula (VI)

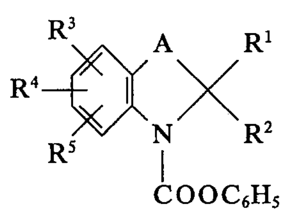

(VI)

in which

A and $R^1$ to $R^5$ possess the abovementioned meaning,

is reacted direct, or after it has been isolated, with an amine of the general formula (VII)

$$H_2N-R \quad (VII)$$

or with a diamine of the general formula (VIII)

$$H_2N-(CH_2)_{n1}-(X)_l-(CH_2)_{n2}-NH_2 \qquad (VIII)$$

in which

R, $n_1$, $n_2$ and l    have the abovementioned meanings,

in an inert organic solvent at temperatures between 20 and 140° C.

7. Medicaments which influence the lipometabolism and contain at least one compound according to Claim 1 or 2.

8. Process for the preparation of medicaments which influence the lipometabolism, characterised in that compounds according to Claim 1 or 2 are converted to a suitable administration form, optionally using conventional auxiliaries and excipients.

9. Compounds of the general formula (I) according to Claim 1 for use in the preparation of medicaments.

10. Compounds of the general formula (I) according to Claim 1, for use in the control of hyperlipidaemia.

**Revendications**

1. Alkylurées de formule générale I

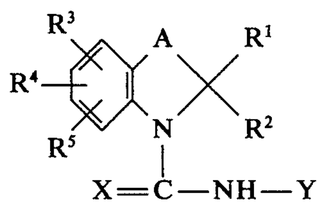

(I)

dans laquelle

Y    représente R ou un reste

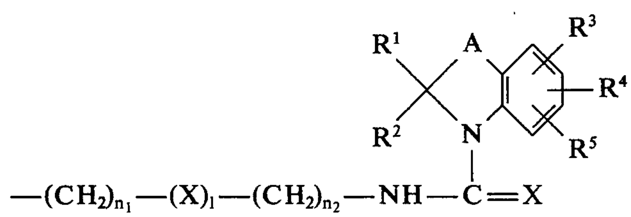

$n_1$ et $n_2$    sont des nombres entiers de 3 à 9 et
l    est égal à 0 ou 1,

R    représente un reste hydrocarboné aliphatique à chaîne droite, ramifié, cyclique, saturé ou insaturé contenant de 4 à 20 atomes de carbone, qui est éventuellement substitué par des halogènes, des groupes hydroxy, alcoxy ou alcoxycarbonyle contenant chacun 1 à 6 atomes de carbone dans le reste alcoxy, ou par un groupe phényle, le groupe phényle portant lui-même éventuellement 1 ou 2 substituants choisis parmi les halogènes, les groupes trifluorométhyle, hydroxy, alkyle en C1 – C2 ou alcoxy en C1 – C2,

$R^1$    représente l'hydrogène ou un groupe alkyle en C1 – C4,

$R^2$    représente un groupe alkyle en C1 – C4,

$R^3$, $R^4$ et $R^5$,    identiques ou différents, représentent chacun l'hydrogène, un halogène, un groupe hydroxy, cyano, trifluorométhyle, alkyle, alkylmercapto, alkylsulfonyle, alkylsulfinyle, alkylcarbonyle et alcoxycarbonyle, les restes alkyle et alcoxy mentionnés contenant chacun 1 à 4 atomes de carbone, ou un groupe phényle ou phénoxy, les restes phényle étant éventuellement substitués une ou deux fois par des halogènes, des groupes trifluorométhyle ou alcoxy en C1 – C2,

X    représente l'oxygène ou le soufre et

A    représente l'un des restes suivants:

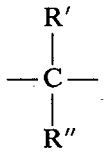 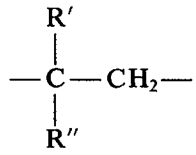 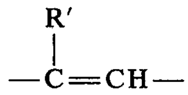 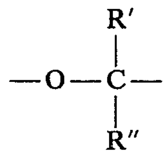 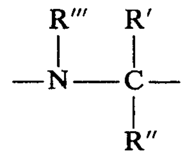

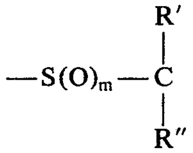

dans lesquels

| | |
|---|---|
| R′ et R″ | identiques ou différents, représentent chacun l'hydrogène ou un groupe alkyle en C1 – C4, |
| R‴ | représente un groupe alkyle en C1 – C4 et |
| m | est égal à 0 ou 2, |

pour l'utilisation dans le traitement des maladies du métabolisme des matières grasses.

2. Dérivés d'alkylurées de formule générale I de la revendication 1, dans laquelle

| | |
|---|---|
| R | représente un groupe alkyle ou alcényle à chaîne droite, ramifié ou cyclique contenant de 6 à 18 atomes de carbones, les groupes alkyle et alcényle mentionnés étant éventuellement substitués par le chlore, le brome, le fluor ou un groupe alcoxy en C1 – C4 ou par un groupe phényle, |
| $R^1$ et $R^2$ | identiques ou différentes, représentent chacun un groupe alkyle en C1 ou C2, |
| $R^3$, $R^4$ et $R^5$ | identiques ou différents, représentent chacun l'hydrogène, le chlore, le brome, un groupe trifluorométhyle, alkyle ou alkylmercapto, les restes alkyle mentionnés contenant chacun 1 à 4 atomes de carbone, ou un groupe phénoxy ou chlorophénoxy, |
| A | représente l'un des restes suivants |

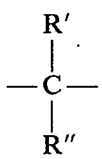 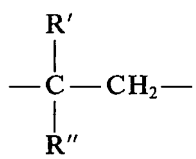 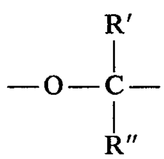 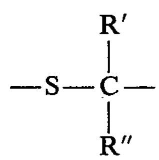

dans lesquels

| | |
|---|---|
| R′ et R″ | identiques ou différents, représentent chacun l'hydrogène, un groupe méthyle ou éthyle et |
| X | représente l'oxygène. |

3. Dérivés d'alkylurées selon la revendication 2, caractérisés en ce que

| | |
|---|---|
| R | représente un groupe alkyle ou alcényle à chaîne droite, ramifié ou cyclique contenant chacun 6 à 18 atomes de carbone, et éventuellement substitué par le chlore, le brome, le fluor ou un groupe alcoxy en C1 – C4, |
| $R^1$ et $R^2$ | identiques ou différents, représentent chacun un groupe méthyle ou éthyle, |
| $R^3$, $R^4$ et $R^5$ | identiques ou différents, représentent chacun l'hydrogène, le chlore, le brome, un groupe trifluorométhyle, phénoxy, chlorophénoxy, alkyle ou mercaptoalkyle contenant chacun 1 ou 2 atomes de carbone, |
| X | représente l'oxygène et |
| A | représente l'un fes restes suivants |

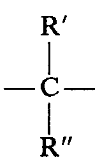 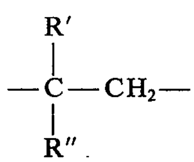 $—OCH_2—$ $—SCH_2—$

dans lesquels

R′ et R″ représentent chacun un groupe méthyle ou éthyle.

4. La N-n-dodécylaminocarbonyl-2,2,4-triméthyl-1,2,3,4-tétrahydroquinoléine.

5. Dérivés d'alkylurées de formule générale I selon la revendication 1, dans laquelle

Y représente un reste

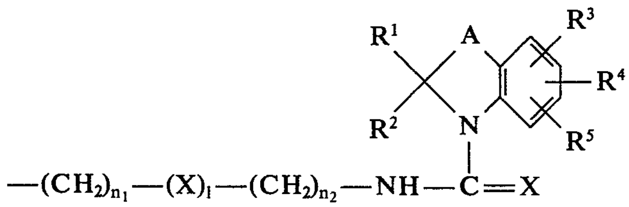

$n_1$ et $n_2$ sont des nombres entiers de 3 à 9 et
l est égal à 0 ou 1, et
$R^1$, $R^2$, $R^3$, $R^4$, $R^5$, A et X ont les significations indiquées dans les revendications 2 ou 3.

6. Procédé de préparation des composés de formule générale I selon les revendications 1 et 3, caractérisé en ce que

a) on fait réagir une amine de formule générale II

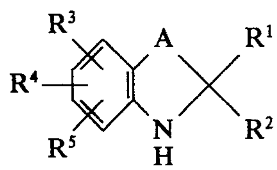

(II)

dans laquelle

A et $R^1$ à $R^5$ ont les significations indiquées ci-dessus,

avec un composé de formule générale III

$$R-N=C=X \qquad (III)$$

ou respectivement un composé de formule générale IV

$$X=C=N-(CH_2)_{n_1}-(X)_l-(CH_2)_{n_2}-N=C=X \qquad (IV)$$

R, X, $n_1$, $n_2$ et l ayant les significations indiquées ci-dessus,

éventuellement dans un solvant organique inerte et éventuellement en présence d'un catalyseur, à des températures de 20 à 120° C, ou bien

b) on fait réagir une amine de formule générale II avec le chloroformiate de phényle de formule V

$$ClCOOC_6H_5 \qquad (V)$$

à des températures de 0 à 50° C, formant ainsi l'ester phénylcarbamique de formule générale VI

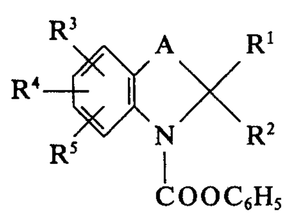

(VI)

dans laquelle

A et $R^1$ à $R^5$ ont les significations indiquées ci-dessus,

qu'on fait réagir directement ou après l'avoir isolé, avec une amine de formule générale VII

$$H_2N-R \quad (VII)$$

ou respectivement une diamine de formule générale VIII

$$H_2N-(CH_2)_{n1}-(X)_l-(CH_2)_{n2}-NH_2 \quad (VIII)$$

R, $n_1$ et l ayant les significations indiquées ci-dessus

dans un solvant organique inerte à des températures de 20 à 140° C.

7. Médicaments agissant sur le métabolisme des matières grasses, contenant au moins un composé selon la revendication 1 ou 2.

8. Procédé de préparation de médicaments agissant sur le métabolisme des matières grasses, caractérisé en ce que l'on met des composés selon la revendication 1 ou 2 sous une forme d'administration appropriée, éventuellement avec utilisation de véhicules et produits auxiliaires usuels.

9. Composés de formule générale I selon la revendication 1, pour l'utilisation dans la préparation de médicaments.

10. Composés de formule générale I selon la revendication 1, pour l'utilisation dans le traitement de l'hyperlipidémie.